(19) Europäisches Patentamt
European Patent Office
Office européen des brevets

(11) Veröffentlichungsnummer: **0 059 388**
**B1**

(12) # EUROPÄISCHE PATENTSCHRIFT

(45) Veröffentlichungstag der Patentschrift: **29.05.85**

(21) Anmeldenummer: **82101270.5**

(22) Anmeldetag: **19.02.82**

(51) Int. Cl.⁴: **C 07 D 471/14,** C 07 D 471/04, A 61 K 31/55 // (C07D471/14, 243:00, 235:00, 221:00),(C07D471/04, 243:00, 221:00)

(54) **Imidazodiazepine, Verfahren und Zwischenprodukte zu deren Herstellung, sowie diese enthaltende Arzneimittel.**

(30) Priorität: **27.02.81 CH 1342/81**

(43) Veröffentlichungstag der Anmeldung: **08.09.82 Patentblatt 82/36**

(45) Bekanntmachung des Hinweises auf die Patenterteilung: **29.05.85 Patentblatt 85/22**

(84) Benannte Vertragsstaaten: **BE CH DE FR GB IT LI NL SE**

(56) Entgegenhaltungen:
**DE - A - 2 416 608**
**DE - A - 2 649 116**

(73) Patentinhaber: **F. HOFFMANN-LA ROCHE & CO. Aktiengesellschaft, CH-4002 Basel (CH)**

(72) Erfinder: **Hunkeler, Walter, Dr., Im Stigler 32, CH-4465 Magden (CH)**
Erfinder: **Kyburz, Emilio, Dr., Unterer Rebbergweg 127, CH-4153 Reinach (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 059 388 B1

**Beschreibung**

Die vorliegende Erfindung betrifft Imidazodiazepine. Im speziellen betrifft sie Imidazodiazepine der allgemeinen Formel

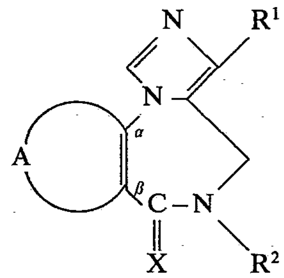

(I)

worin A zusammen mit den beiden mit α und β bezeichneten Kohlenstoffatomen einen Pyridinring, X ein Sauerstoff- oder Schwefelatom, $R^1$ Wasserstoff, Halogen, niederes Alkyl, niederes Alkoxymethyl oder die Gruppe $-COOR^3$ mit $R^2$ und $R^3$ je niederes Alkyl bedeuten,
und pharmazeutisch annehmbare Säureadditionssalze davon.

Diese Verbindungen sind neu und zeichnen sich durch wertvolle pharmakodynamische Eigenschaften aus. Sie eignen sich demnach zur Verwendung bei der Bekämpfung bzw. Verhütung von Krankheiten.

Gegenstand der vorliegenden Erfindung sind Verbindungen der allgemeinen Formel I und pharmazeutisch annehmbare Säureadditionssalze davon als solche und als pharmazeutische Wirkstoffe, die Herstellung dieser Verbindungen, und Zwischenprodukte für die Herstellung dieser Verbindungen, ferner Arzneimittel, enthaltend eine Verbindung der allgemeinen Formel I oder ein pharmazeutisch annehmbares Säureadditionssalz davon, und die Herstellung solcher Arzneimittel.

Das Symbol A bezeichnet zusammen mit den beiden mit α und β bezeichneten Kohlenstoffatomen die Gruppe

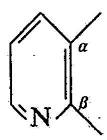 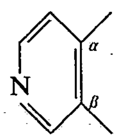 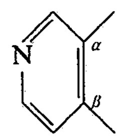 oder 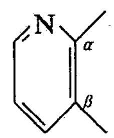

(a) (b) (c) (d)

Der Ausdruck »niederes Alkyl« bezeichnet gesättigte Kohlenwasserstoffreste, welche geradkettig oder verzweigt sein können, mit höchstens 7, vorzugsweise höchstens 4 Kohlenstoffatomen, wie Methyl, Äthyl, Isopropyl, t-Butyl und dergleichen. Der Ausdruck »niederes Alkoxymethyl« umfaßt Reste wie Methoxymethyl, Äthoxymethyl und dergleichen. Der Ausdruck »Halogen« bedeutet die vier Formen Fluor, Chlor, Brom und Jod.

$R^1$ bedeutet vorzugsweise Chlor oder die Gruppe $-COOR^3$, wobei $R^3$ vorzugsweise Methyl, Äthyl, Isopropyl oder t-Butyl bedeutet. In einer besonders bevorzugten Ausführungsform bedeuten $R^1$ die Gruppe $-COOR^3$ und $R^3$ Äthyl oder t-Butyl.

Das Symbol A bedeutet vorzugsweise die Gruppe (a), (b) oder (d) und besonders bevorzugt die Gruppe (b). Die bevorzugte Bedeutungsmöglichkeit von $R^2$ ist Methyl. Das Symbol X bedeutet vorzugsweise ein Sauerstoffatom.

Eine ganz besonders bevorzugte Verbindung im Rahmen der vorliegenden Erfindung ist t-Butyl-5,6-dihydro-5-methyl-6-oxo-4H-imidazo[1,5-a]pyrido[3,4-f] [1,4]diazepin-3-carboxylat.

Weitere im Rahmen der vorliegenden Erfindung besonders bevorzugte von der allgemeinen Formel I umfaßte Verbindungen sind:

t-Butyl-5,6-dihydro-5-methyl-6-oxo-4H-imidazo[1,5-a]pyrido[2,3-f] [1,4]diazepin-3-carboxylat,
Äthyl-5,6-dihydro-5-methyl-6-oxo-4H-imidazo[1,5-a]pyrido[2,3-f] [1,4]diazepin-3-carboxylat,
3-Chlor-4,5-dihydro-5-methyl-6H-imidazo[1,5-a]pyrido[2,3-f] [1,4]diazepin-6-on,
t-Butyl-5,6-dihydro-5-methyl-6-oxo-4H-imidazo[1,5-a]pyrido[3,2-f] [1,4]diazepin-3-carboxylat und
Äthyl-5,6-dihydro-5-methyl-6-oxo-4H-imidazo[1,5-a]pyrido[3,4-f] [1,4]diazepin-3-carboxylat.

Die Imidazodiazepine der allgemeinen Formel I und ihre pharmazeutisch annehmbaren Säureadditionssalze können erfindungsgemäß hergestellt werden, indem man

a) eine Verbindung der allgemeinen Formel

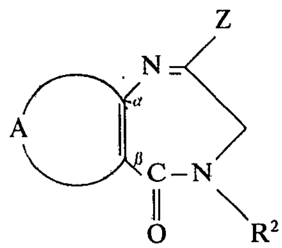

(II)

worin A und $R^2$ obige Bedeutung besitzen, und Z eine Abgangsgruppe bedeutet, in Gegenwart einer Base mit einem Isocyanessigester der allgemeinen Formel

$$CN-CH_2-COOR^3 \qquad (III)$$

worin $R^3$ obige Bedeutung besitzt, umsetzt, oder

b) eine Carbonsäure der allgemeinen Formel

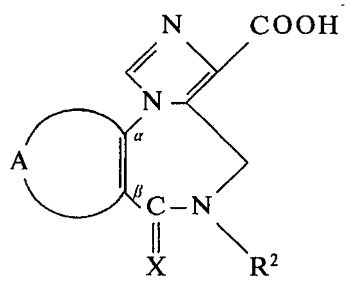

(IV)

worin A, X und $R^2$ obige Bedeutung besitzen, decarboxyliert, oder

c) eine Verbindung der allgemeinen Formel

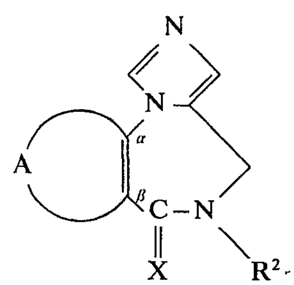

(Ia)

worin A, X und $R^2$ obige Bedeutung besitzen, halogeniert, oder

d) eine Verbindung der allgemeinen Formel

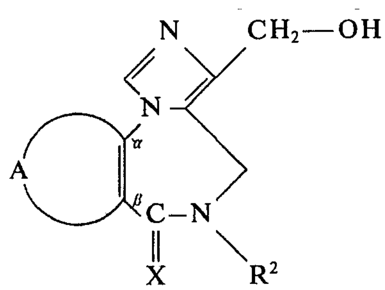

(Va)

bzw.

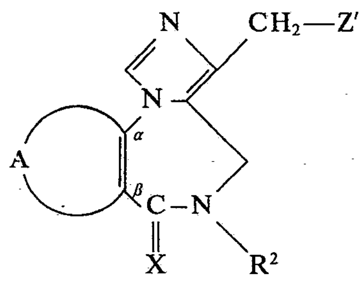

(VIa)

worin A, X und $R^2$ obige Bedeutung besitzen und Z′ eine Abgangsgruppe bedeutet, mit einem einen niederen Alkylrest liefernden Alkylierungsmittel, bzw. mit einem niederen Alkohol veräthert, oder

e) in einer Verbindung der allgemeinen Formel

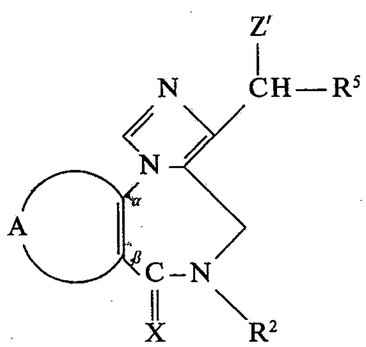

(VI)

worin A, X, Z′ und $R^2$ obige Bedeutung besitzen und $R^5$ Wasserstoff oder niederes Alkyl bedeutet, die mit Z′ bezeichnete Abgangsgruppe unter reduktiven Bedingungen abspaltet, oder

f) eine Verbindung der allgemeinen Formel

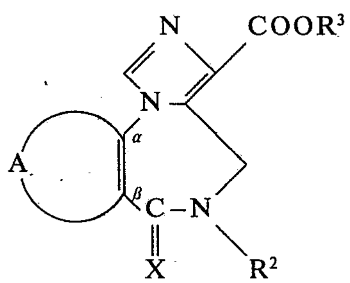

(Ic)

worin A, X, $R^2$ und $R^3$ obige Bedeutung besitzen, umestert, oder

g) in einer Verbindung der allgemeinen Formel

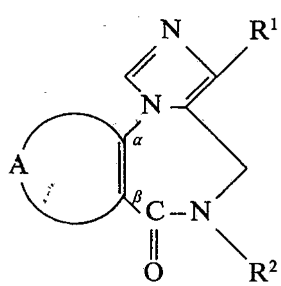

(Ib)

worin A, $R^1$ und $R^2$ obige Bedeutung besitzen, die Carbonylgruppe in die Thiocarbonylgruppe überführt, und

h) erwünschtenfalls eine erhaltene Verbindung der allgemeinen Formel I in ein pharmazeutisch annehmbares Säureadditionssalz überführt.

Gemäß Verfahrensvariante a) können Verbindungen der allgemeinen Formel I demnach aus Verbindungen der allgemeinen Formel II und Isocyanessigestern der allgemeinen Formel III hergestellt werden. Die in Formel II mit Z bezeichnete Abgangsgruppe ist beispielsweise eine leicht abspaltbare Phosphinylgruppe, z. B. eine Gruppe der Formel

$$-\overset{\overset{\displaystyle O}{\|}}{O P}(OR^7)_2 \quad \text{oder} \quad -\overset{\overset{\displaystyle O}{\|}}{O P}(NR^8R^9)_2$$

worin $R^7$ niederes Alkyl und $R^8$ und $R^9$ je niederes Alkyl, Allyl, Phenyl oder substituiertes Phenyl oder zusammen mit dem Stickstoffatom einen unsubstituierten oder substituierten heterocyclischen Ring mit 3—8 Gliedern (wie Morpholin) bedeuten,
ein Halogenatom, eine Alkylthiogruppe, eine Aralkylthiogruppe, eine N-Nitrosoalkylaminogruppe, eine Alkoxygruppe, eine Mercaptogruppe und dergleichen (wenn Z eine Mercaptogruppe bedeutet, so handelt es sich bei der entsprechenden Verbindung der Formel II um die Iminothiol-Form des entsprechenden Thiolactams). Die Umsetzung der Verbindungen der Formeln II und III erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um das Anion des Isocyanessigesters der Formel III zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmäßigerweise zwischen etwa —40° C und etwa Raumtemperatur.

Gemäß Verfahrensvariante b) können Verbindungen der allgemeinen Formel I, worin $R^1$ Wasserstoff bedeutet, durch Decarboxylierung von Carbonsäuren der allgemeinen Formel IV hergestellt werden. Diese Reaktion erfolgt vorzugsweise durch trockenes Erhitzen der gegebenenfalls rohen Carbonsäure der allgemeinen Formel IV auf Temperaturen von etwa 150° C bis etwa 400° C, wobei die Reaktionstemperatur vom Schmelzpunkt der jeweils eingesetzten Verbindung der Formel IV abhängt.

Gemäß Verfahrensvariante c) können Verbindungen der allgemeinen Formel I, worin $R^1$ Halogen bedeutet, durch Halogenierung von Verbindungen der allgemeinen Formel Ia hergestellt werden. Geeignete Halogenierungsmittel sind beispielsweise N-Chlorsuccinimid, N-Bromsuccinimid, N-Chloracetamid, N-Bromacetamid und elementares Jod. Als Lösungsmittel verwendet man zweckmäßigerweise inerte organische Lösungsmittel, beispielsweise halogenierte Kohlenwasserstoffe, wie Methylenchlorid, 1,2-Dichloräthan, Chloroform und dergleichen, Dimethylformamid, Dimethylacetamid, Acetonitril, Äther, wie Diäthyläther, Tetrahydrofuran, Dioxan und dergleichen, usw. Die Halogenierung kann je nach eingesetztem Lösungsmittel in einem Temperaturbereich von etwa 0° C bis etwa 120° C durchgeführt werden.

Gemäß Verfahrensvariante d) können Verbindungen der allgemeinen Formel I, worin $R^1$ niederes Alkoxymethyl bedeutet, hergestellt werden, indem man einen Alkohol der allgemeinen Formel Va mit einem den gewünschten niederen Alkylrest liefernden Alkylierungsmittel, oder eine Verbindung der allgemeinen Formel VIa mit einem niederen Alkohol veräthert. Diese Reaktion erfolgt in einem inerten organischen Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um aus dem Alkohol der allgemeinen Formel Va, bzw. aus dem niederen Alkohol das entsprechende Alkoholat zu bilden. Als Basen eignen sich z. B. Alkalimetallhydride, wie Natriumhydrid, Alkalimetalle, wie Natrium, und Alkalimetallamide, wie Lithiumamid und Lithiumdiisopropylamid. Geeignete Alkylierungsmittel sind beispielsweise Halogenide, wie Methyljodid, Äthylbromid und Äthyljodid, und Dialkylsulfate, wie Dimethylsulfat und Diäthylsulfat. Die Reaktionstemperatur liegt zweckmäßigerweise zwischen etwa 0° C und etwa 50° C.

Gemäß Verfahrensvariante e) können Verbindungen der allgemeinen Formel I, worin $R^1$ niederes Alkyl bedeutet, hergestellt werden, indem man in einer Verbindung der allgemeinen Formel VI die mit Z′ bezeichnete Abgangsgruppe unter reduktiven Bedingungen abspaltet. Diese Reaktion erfolgt nach an sich bekannten Methoden, wobei die Wahl der geeigneten Abgangsgruppe Z′ sowie die Ermittlung der für die Abspaltung geeigneten Reaktionsbedingungen, unter welchen andere im Molekül enthaltene Strukturelemente nicht angegriffen werden dürfen, dem Fachmann keinerlei Schwierigkeiten bereiten. Für den vorliegenden Verfahrensaspekt besonders geeignete Abgangsgruppen sind beispielsweise Halogenatome, wie Chlor, Brom und Jod, welche sich unter hydrogenolytischen Bedingungen leicht abspalten lassen, beispielsweise durch Behandeln mit elementarem Wasserstoff in Gegenwart eines geeigneten Katalysators (z. B. Palladium/Kohle, Raney-Nickel, etc.) in einem inerten organischen Lösungsmittel. Geeignete Lösungsmittel sind beispielsweise Alkohole, wie Methanol, Äthanol und Isopropanol, Äther, wie Diäthyläther, Tetrahydrofuran, Dioxan und Dimethoxyäthan, und dergleichen. Je nach Reaktivität des eingesetzten Katalysators arbeitet man bei Drucken von etwa Normaldruck bis etwa 300 bar und bei Temperaturen von etwa Raumtemperatur bis etwa 150° C.

Gemäß Verfahrensvariante f) können Verbindungen der allgemeinen Formel I durch Umesterung von Verbindungen der allgemeinen Formel Ic hergestellt werden, d. h. daß in Verbindungen der

allgemeinen Formel Ic der mit $R^3$ bezeichnete Alkylrest gegen einen anderen Rest $R^3$ ausgetauscht wird.

Diese Umesterung erfolgt in an sich bekannter Weise durch Umsetzen einer Verbindung der allgemeinen Formel Ic mit einem dem gewünschten Rest $R^3$ entsprechenden Alkohol, d. h. z. B. mit Methanol, Äthanol oder Isopropanol, bei Raumtemperatur oder unter Erwärmen auf eine Temperatur von etwa 25° bis 150°C. Vorzugsweise wird die Umesterung in Gegenwart einer Base durchgeführt, wobei sich im vorliegenden Fall insbesondere Kaliumcyanid oder ähnliche schwache Basen eignen. Als Base eignet sich aber auch das dem gewünschten Rest $R^3$ entsprechende Alkoholat, d. h. z. B. Natriummethanolat, -äthanolat oder -isopropanolat, oder die entsprechenden Kaliumsalze. Als Lösungsmittel dient vorzugsweise der dem Rest $R^3$ im gewünschten Endprodukt der Formel I entsprechende Alkohol; die Umesterung kann jedoch auch in einem inerten organischen Lösungsmittel erfolgen, beispielsweise in einem aromatischen Kohlenwasserstoff, wie Benzol oder Xylol, einem Äther, wie Dioxan, Tetrahydrofuran oder Äthylenglykol-dimethyläther, in Dimethylformamid, in Dimethylsulfoxid oder dergleichen. Bei dieser Umesterung kann man nicht nur einen niedriger siedenden Alkohol durch einen höher siedenden Alkohol, sondern auch einen höher siedenden Alkohol durch einen niedriger siedenden Alkohol ersetzen.

Die Umesterung kann indessen ohne weiteres auch mehrstufig durchgeführt werden, beispielsweise dadurch, daß man die eingesetzte Verbindung der Formel Ic (wie weiter unten beschrieben) zur entsprechenden freien Carbonsäure der Formel IV hydrolisiert, aus dieser ein reaktionsfähiges funktionelles Derivat (z. B. ein Säurechlorid oder dergleichen) herstellt und anschließend dieses reaktionsfähige Carbonsäurederivat mit dem der Bedeutung von $R^3$ in der gewünschten Verbindung der Formel I entsprechenden Alkohol zur Reaktion bringt. Dieses Vorgehen drängt sich insbesondere dann auf, wenn ein t-Butylester gewünscht wird, oder wenn $R^3$ in einer Verbindung der Formel Ic t-Butyl bedeutet.

Gemäß Verfahrensvariante g) können Verbindungen der allgemeinen Formel Ib in entsprechende Verbindungen der Formel I, worin X ein Schwefelatom bedeutet, übergeführt werden, und zwar durch Behandeln mit einem Schwefelungsmittel, was in an sich bekannter Weise erfolgen kann. Beispielsweise kann man als Schwefelungsmittel Phosphorpentasulfid verwenden, wobei dieses vorzugsweise im Überschuß eingesetzt wird und die Reaktion vorteilhafterweise in einem inerten organischen Lösungsmittel, wie Dioxan, Methylenchlorid oder dergleichen, in Gegenwart von Triäthylamin bei einer Temperatur von etwa 50°C bis zur Rückflußtemperatur des Reaktionsgemisches erfolgt. Als Schwefelungsmittel eignen sich indessen auch Verbindungen wie 2,4-Bis-(p-methoxyphenyl)-1,3,2,4-dithiaphosphetan-2,4-disulfid; derartige Schwefelungsmittel werden ungefähr in der berechneten Menge eingesetzt, und die Reaktion erfolgt in Gegenwart eines inerten Lösungsmittels, wie Toluol, Xylol, zweckmäßigerweise bei Rückflußtemperatur des Reaktionsgemisches oder in Hexamethyl-phosphorsäuretriamid zwischen etwa 60 und 110°C.

Gemäß Verfahrensvariante h) können Verbindungen der allgemeinen Formel I erfindungsgemäß in pharmazeutisch annehmbare Säureadditionssalze übergeführt werden. Die Herstellung von solchen pharmazeutisch annehmbaren Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der allgemeinen Formel II können ausgehend von Verbindungen der Formel

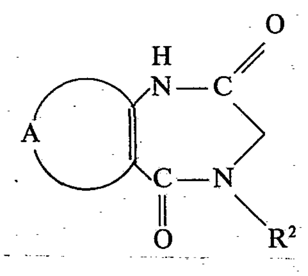

(VIII)

worin A und $R^2$ obige Bedeutung besitzen,
hergestellt werden, und zwar nach Methoden, welche an sich bekannt sind, vgl. z. B. die Belgischen Patentschriften Nr. 802 233, 833 249 und 865 653, die US-Patentschrift Nr. 3 681 341 und J. Org. Chemistry 29, 231 (1964).

Verschiedene der weiter unten folgenden Beispiele enthalten detaillierte Angaben betreffend die Herstellung von Verbindungen der allgemeinen Formel II aus Verbindungen der allgemeinen Formel VIII.

Die Verbindungen der Formel VIII ihrerseits können nach an sich bekannten Methoden leicht hergestellt werden, beispielsweise gemäß nachfolgendem Formelschema 1, worin A′ die Gruppe (a) oder (b) und A″ die Gruppe (c) oder (d) bedeuten und $R^2$ obige Bedeutung besitzt:

Formelschema 1

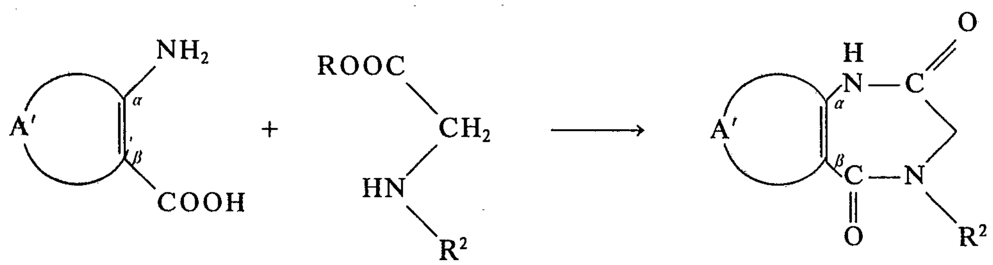

(IX) (X) (VIIIa)

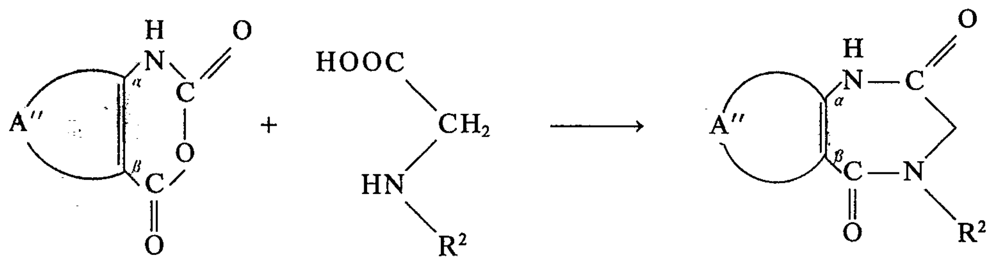

(XI) (XII) (VIIIb)

Die Reaktion einer Verbindung der Formel IX mit einer Verbindung der Formel X erfolgt in Gegenwart von N,N'-Carbonyldiimidazol in einem inerten organischen Lösungsmittel, wie Dimethylformamid, Tetrahydrofuran, Dioxan oder dergleichen. Die Reaktion wird vorzugsweise in einem »Eintopfverfahren« durchgeführt, d. h. daß man das in einem ersten Schritt gebildete Carbonsäureimidazolid nicht isoliert, sondern direkt mit einer Verbindung der Formel X zu einer Verbindung der Formel VIIIa umsetzt.

Die Reaktion einer Verbindung der Formel XI mit einer Aminosäure der Formel XII zu einer Verbindung der Formel VIIIb erfolgt in einem inerten organischen Lösungsmittel, wie Dimethylsulfoxid, Dimethylformamid oder dergleichen, in einem Temperaturbereich von etwa 50° C bis etwa 200° C.

Die als Ausgangsstoffe verwendeten Carbonsäuren der allgemeinen Formel IV können hergestellt werden, indem man in einer Verbindung der allgemeinen Formel Ic die Estergruppe hydrolysiert. Diese Reaktion erfolgt nach an sich bekannten und jedem Fachmann geläufigen Methoden. Handelt es sich bei der Verbindung der allgemeinen Formel Ic um einen tertiären Alkylester, z. B. um einen t-Butylester, so wird die Hydrolyse zweckmäßigerweise in saurer Lösung durchgeführt. Als Säuren kommen dabei Trifluoressigsäure, konzentrierte oder verdünnte Mineralsäuren, wie Salzsäure, Bromwasserstoffsäure, Phosphorsäure und dergleichen, usw. in Betracht. Die Reaktion erfolgt zweckmäßigerweise bei erhöhter Temperatur, beispielsweise bei der Siedetemperatur des Reaktionsgemisches oder wenig darunter. In den übrigen Fällen wird die obige Hydrolyse zweckmäßigerweise mit einer wäßrigen Lauge, wie Natronlauge und Kalilauge, gegebenenfalls in Gegenwart eines Lösungsvermittlers, durchgeführt. Als Lösungsvermittler eignen sich Alkohole, wie Methanol und Äthanol, Äther, wie Tetrahydrofuran und Dioxan, und dergleichen. Die alkalische Hydrolyse erfolgt vorzugsweise in einem Temperaturbereich von etwa Raumtemperatur bis Siedetemperatur des Reaktionsgemisches.

Die als Ausgangsstoffe verwendeten Verbindungen der allgemeinen Formel Va können beispielsweise durch Reduktion von Carbonsäureestern der allgemeinen Formel Ic hergestellt werden. Die Reduktion erfolgt vorzugsweise mit einem Reduktionsmittel, wie Lithiumborhydrid, in einem inerten organischen Lösungsmittel, wie Diäthyläther, Tetrahydrofuran, Dimethoxyäthan oder dergleichen.

Die als Ausgangsstoffe verwendeten Verbindungen der Formel VI sind aus Verbindungen der Formel Va, gemäß nachfolgendem Formelschema 2, worin A, X, Z' und $R^2$ obige Bedeutung besitzen und $R^{51}$ niederes Alkyl bedeutet, leicht zugänglich:

Formelschema 2

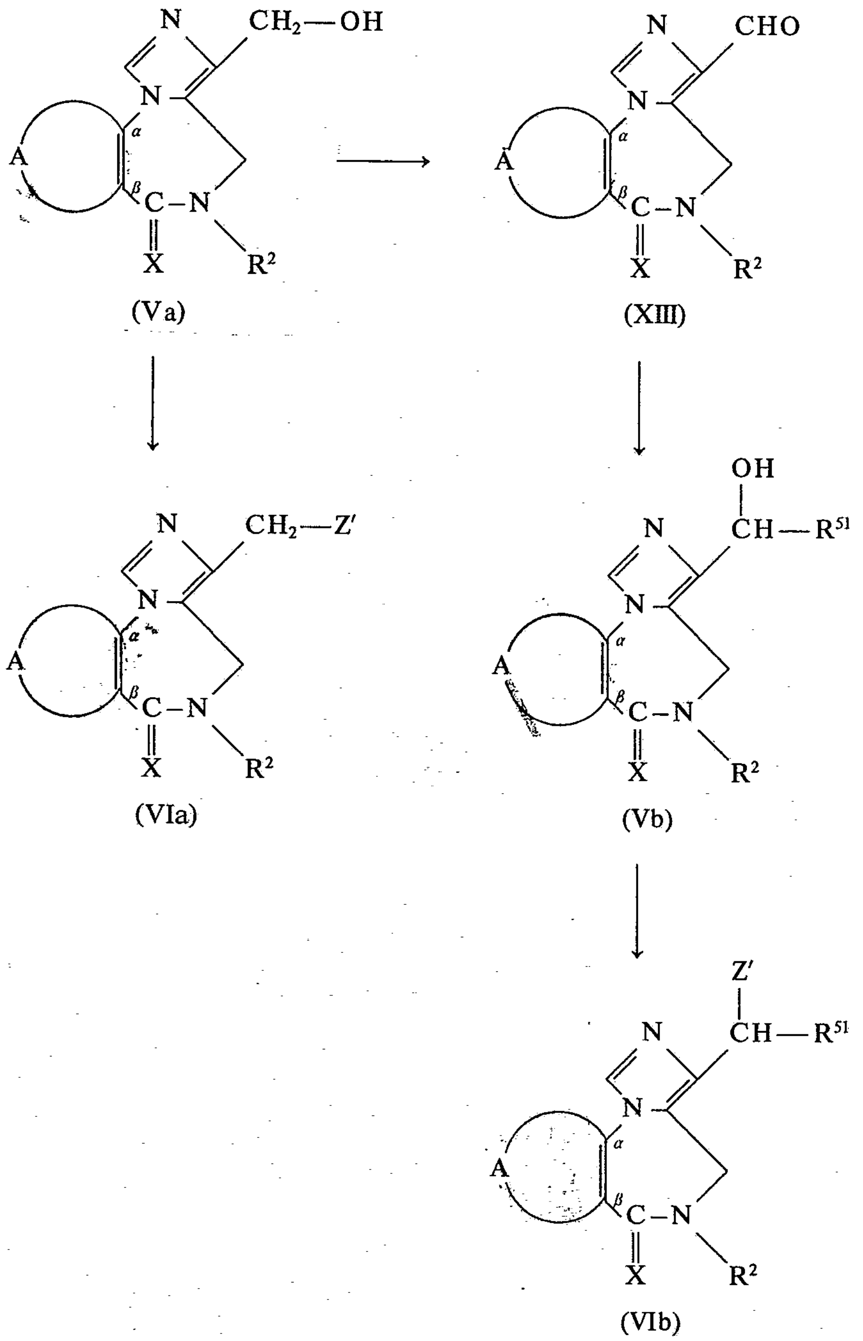

(Va) (XIII) (VIa) (Vb) (VIb)

Die Herstellung einer Verbindung der Formel XIII aus einem Alkohol der Formel Va erfolgt vorzugsweise mit einem milden Oxidationsmittel, wie Mangandioxid oder dergleichen, in einem inerten organischen Lösungsmittel, wie Methylenchlorid, Chloroform oder dergleichen.

Die Verbindungen der Formel Vb können hergestellt werden, indem man eine Verbindung der allgemeinen Formel XIII nach allgemein bekannten und jedem Fachmann geläufigen Methoden mit einer den Rest $R^{51}$ liefernden metallorganischen Verbindung umsetzt. Als metallorganische Verbindungen kommen in erster Linie Grignard-Verbindungen, wie Methylmagnesiumjodid, Äthyl-magnesiumjodid, Isopropyl-magnesiumbromid, n-Propyl-magnesiumbromid, n-Butyl-magnesiumchlorid und dergleichen, in Frage. Als Lösungsmittel eignen sich Äther, wie Diäthyläther, Tetrahydrofuran, t-Butylmethyläther, Mischungen davon, und dergleichen. Zweckmäßigerweise arbeitet man bei der Siedetemperatur des Reaktionsgemisches, man kann aber auch unterhalb davon arbeiten, beispielsweise bei Raumtemperatur.

Die Verbindungen der allgemeinen Formel VI, d. h. VIa und VIb, können nach allgemein bekannten und jedem Fachmann geläufigen Methoden aus Verbindungen der Formel Va bzw. Vb hergestellt werden. Entsprechende Halogenide erhält man beispielsweise durch Behandeln von Verbindungen

der Formel Va bzw. Vb mit Halogenierungsmitteln, wie Thionylchlorid, Phosphoroxychlorid, Phosphorpentachlorid, Tetrabromkohlenstoff/Triphenylphosphin und dergleichen.

Die als Ausgangsstoffe verwendeten Verbindungen der allgemeinen Formeln II, IV, Va und VI sind neu und ebenfalls Gegenstand der vorliegenden Erfindung.

Wie eingangs erwähnt, sind die Verbindungen der allgemeinen Formel I neu und besitzen äußerst wertvolle pharmakodynamische Eigenschaften. Sie weisen nur eine geringe Toxizität auf, und es hat sich gezeigt, daß sie eine ausgeprägte Affinität zu den zentralen Benzodiazepin-Rezeptoren haben und die zentral dämpfenden, muskelrelaxierenden, ataktischen, blutdrucksenkenden und atemdepressiven Eigenschaften von tranquilisierend wirksamen 1,4-Benzodiazepinen zu antagonisieren vermögen.

Die Affinität von Verbindungen der allgemeinen Formel I zu den zentralen Benzodiazepin-Rezeptoren wurde nach der in Life Science 20, 2101—2110 (1977) und Science 198, 849—851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ (»50% inhibiting concentration«) diejenige Konzentration der jeweiligen Testsubstanz, welche eine 50-proz. Hemmung der spezifischen Bindung des tritiierten Diazepams an die spezifischen Benzodiazepin-Rezeptoren im cerebralen Cortex bewirkt.

Eine der typischen Eigenschaften von tranquilisierend wirksamen 1,4-Benzodiazepinen in Versuchstieren ist ihre ausgeprägte anticonvulsive Wirkung, welche beispielsweise im bekannten und allgemein anerkannten Pentetrazol-Test nachgewiesen werden kann. Diese Eigenschaft wurde verwendet, um den nachstehend beschriebenen Test auszuarbeiten, der es erlaubt, Verbindungen zu ermitteln, die die zentralen Eigenschaften von tranquilisierend wirksamen 1,4-Benzodiazepinen zu antagonisieren vermögen.

In diesem Test verabreicht man Mäusen eine Stunde vor dem Pentetrazol (120 mg/kg, i. p.) 5 mg/kg (i. p.) Diazepam (d. h. eine supramaximale Dosis, die im Pentetrazol-Test an mehr als 900 Mäusen alle Versuchstiere vor krampfartigen Anfällen schützte) und 15 Minuten vor dem Pentetrazol die zu testende Verbindung p. o. oder i. v. Die antagonistische Wirksamkeit der untersuchten Verbindungen, d. h. ihr Vermögen, die Wirkung des Diazepams im Pentetrazol-Test aufzuheben, wird ermittelt, indem man die Mäuse auszählt, die in diesem Test krampfartige Anfälle erleiden.

In der nachstehenden Tabelle werden die Resultate dargestellt, welche mit repräsentativen Vertretern der durch die allgemeine Formel I definierten Verbindungsklasse in dem vorstehend geschilderten Versuch erhalten worden sind. Angegeben wird für jede der darin figurierenden Verbindung der $ED_{50}$-Wert. Als $ED_{50}$ bezeichnet man die Menge der jeweiligen Testverbindung in mg/kg (p. o. oder i. v.), die bei 50% der Tiere den Diazepam-Effekt in obigem Versuch aufhebt. Außerdem enthält die Tabelle die oben definierten $IC_{50}$-Werte für alle darin angegebenen Testverbindungen.

Tabelle

| Verbindung der Formel I, worin | | | | | $IC_{50}$ in | $ED_{50}$ in mg/kg | |
|---|---|---|---|---|---|---|---|
| A | $R^1$ | $R^2$ | $R^3$ | X | nM/1 | p. o. | i. v. |
| (b) | $-COOC(CH_3)_3$ | H | $-CH_3$ | 0 | 3,0 | 0,23 | 0,16 |
| (b) | $-COOCH_2CH_3$ | H | $-CH_3$ | 0 | 7,0 | 1,94 | — |
| (a) | $-COOC(CH_3)_3$ | H | $-CH_3$ | 0 | 17,0 | 5,4 | 3,1 |
| (d) | $-COOC(CH_3)_3$ | H | $-CH_3$ | 0 | 130 | 15,1 | — |

Wie bereits erwähnt, antagonisieren die Verbindungen der Formel I die zentral dämpfenden, muskelrelaxierenden, ataktischen, blutdrucksenkenden und atemdepressiven Eigenschaften von tranquilisierend wirksamen 1,4-Benzodiazepinen. Letztere stehen in der Therapie in vielfältigem Gebrauch und werden oft in hohen Dosierungen angewandt, sodaß die oben erwähnten Wirkungen auch als Nebenwirkungen stark hervortreten können. Die Verbindungen der Formel I können bei Intoxikationen, bei welchen übermäßige Einnahme von tranquilisierend wirksamen 1,4 Benzodiazepinen beteiligt ist, als Antidot verwendet werden. Sie eignen sich auch zur Abkürzung einer durch tranquilisierend wirksame 1,4-Benzodiazepine eingeleiteten Anästhesie in der Chirurgie und in der Geburtshilfe. Bei Neugeborenen kann eine eventuelle Atemdepression, die auf die Gabe von tranquilisierend wirksamen 1,4-Benzodiazepinen an die Mutter zurückgeht, aufgehoben werden. Die Verbindungen der Formel I können auch dazu verwendet werden, bei 1,4-Benzodiazepinen, die in anderen Indikationsgebieten eingesetzt werden, die in einem solchen Fall unerwünschten Wirkungen auf das zentrale Nervensystem zu unterdrücken. Beispiele derartiger, in anderen Indikationsgebieten einsetzbarer 1,4-Benzodiazepine sind die in den britischen Patentschriften Nr. 1 444 529 und 1 474 305 beschriebenen schistosomizid

wirksamen 1,4-Benzodiazepine, wie das (+)-5-(o-Chlorphenyl)-1,3-dihydro-3-methyl-7-nitro-2H-1,4-benzodiazepin-2-on.

Die Verbindungen der allgemeinen Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Trägern verarbeitet werden. Als solche Träger kann man für Tabletten, Dragées und Hartgelatinekapseln z. B. Lactose, Maisstärke oder Derivate davon, Talk, Stearinsäure oder deren Salze etc. verwenden. Für Weichgelatinekapseln eignen sich als Träger z. B. pflanzliche Öle, Wachse, Fette, halbfeste und flüssige Polyole etc. Zur Herstellung von Lösungen und Sirupen eignen sich als Träger z. B. Wasser, Polyole, Saccharose, Invertzucker, Glukose und dergleichen. Für Injektionslösungen eignen sich als Träger z. B. Wasser, Alkohole, Polyole, Glyzerin, pflanzliche Öle etc. Für Suppositorien eignen sich als Träger z. B. natürliche oder gehärtete Öle, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt, kann man Verbindungen der allgemeinen Formel I und pharmazeutisch annehmbare Säureadditionssalze davon bei der Bekämpfung bzw. Verhütung von Krankheiten verwenden, insbesondere bei der Antagonisierung der zentral dämpfenden, muskelrelaxierenden, ataktischen blutdrucksenkenden und atemdepressiven Eigenschaften von tranquilisierend wirksamen 1,4-Benzodiazepinen. Insbesondere kann man Verbindungen der allgemeinen Formel I in Kombination mit den weiter oben erwähnten schistosomizid wirksamen Verbindungen, z. B. in Kombination mit (+)-5-(o-Chlorphenyl)-1,3-dihydro-3-methyl-7-nitro-2H-1,4-benzodiazepin-2-on bei der Bekämpfung der Schistosomiasis verwenden. Dabei können die Verbindungen der Formel I oder deren pharmazeutisch annehmbare Säureadditionssalze vor, gleichzeitig mit oder nach der Verabreichung bzw. Einnahme von tranquilisierend wirksamen 1,4-Benzodiazepinen verabreicht werden. Wird die Verbindung der Formel I oder ein pharmazeutisch annehmbares Säureadditionssalz davon gleichzeitig mit dem tranquilisierend wirksamen 1,4-Benzodiazepin verabreicht, so kann dies durch Verabreichung als ad-hoc-Kombination oder in Form einer pharmazeutischen Kombination erfolgen, welche eine Verbindung der allgemeinen Formel I oder ein pharmazeutisch annehmbares Säureadditionssalz davon und ein tranquilisierend wirksames 1,4-Benzodiazepin-Derivat enthält; derartige pharmazeutische Kombinationen sind ebenfalls Gegenstand der vorliegenden Erfindung. Die Dosierung der Verbindungen der Formel I und ihrer pharmazeutisch annehmbaren Säureadditionssalze kann innerhalb weiter Grenzen variieren und ist natürlich in jedem einzelnen Fall den individuellen Gegebenheiten anzupassen. Im allgemeinen dürfte eine Tagesdosis von etwa 0,2 bis etwa 500 mg angemessen sein.

Wie eingangs erwähnt, sind Arzneimittel, enthaltend eine Verbindung der allgemeinen Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, daß man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Im speziellen sind pharmazeutische Kombinationen, enthaltend eine Verbindung der allgemeinen Formel I und eine der weiter oben erwähnten schistosomizid wirksamen Verbindungen, insbesondere das (+)-5-(o-Chlorphenyl)-1,3-diydro-3-methyl-7-nitro-2H-1,4-benzodiazepin-2-on, Gegenstand der vorliegenden Erfindung; derartige Kombinationen eignen sich zur Bekämpfung von Schistosomiasis.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) 5,52 g (40 mMol) 4-Aminonicotinsäure in 50 ml Dimethylformamid werden mit 6,48 g (40 mMol) N,N'-Carbonyldiimidazol versetzt und während 2,5 Stunden bei 50° gerührt. Zum erhaltenen Reaktionsgemisch gibt man anschließend 4,04 g (40 mMol) Triäthylamin und 6,22 g (40 mMol) Sarcosinmethylester-hydrochlorid und rührt während 2 Stunden bei 80° und während 3,5 Stunden bei der Siedetemperatur. Nach Entfernen des Dimethylformamids im Vakuum wird das erhaltene Rohprodukt durch Säulenchromatographie an Kieselgel und anschließendes Umkristallisieren aus Äthanol gereinigt

Man erhält 3,4-Dihydro-4-methyl-2H-pyrido[4,3-e] [1,4]-diazepin-2,5(1H)-dion vom Schmelzpunkt 267—268°.

b) 3,33 g (17,4 mMol) 3,4-Dihydro-4-methyl-2H-pyrido-[4,3-e] [1,4]diazepin-2,5(1H)-dion in 35 ml Dimethylformamid versetzt man mit 450 mg (18,8 mMol) mit Hexan gewaschener Natriumhydrid-Öldispersion, rührt während 30 Minuten bei Raumtemperatur, tropft anschließend bei —20° 3,0 g (1,4 mMol) Diäthylchlorphosphat zu und rührt während 20 Minuten bei —20°.

Inzwischen wird eine Lösung von 1,96 g (17,5 mMol) Kalium-t-butylat in 4 ml Dimethylformamid auf ca. —50° abgekühlt, mit 2,64 g (17,4 mMol) Isocyanessigsäure-t-butylester versetzt und bei —10° bis —30° dem obigen Reaktionsgemisch zugetropft. Man entfernt die Kühlung, rührt noch während 20 Minuten, gießt auf ca. 200 ml Wasser und extrahiert mit Chloroform. Die vereinigten Chloroformauszüge werden mit Wasser gewaschen, über Magnesiumsulfat getrocknet und eingedampft. Durch Säulenchromatographie an Kieselgel und anschließendes Umkristallisieren aus Essigester erhält man t-Butyl-5,6-dihydro-5-methyl-6-oxo-4H-imidazo[1,5-a]pyrido[3,4-f] [1,4]diazepin-3-carboxylat vom Schmelzpunkt 214—215°.

## Beispiel 2

a) Eine Suspension von 5,18 g (37,5 mMol) 3-Aminopicolinsäure in 100 ml Dimethylformamid wird mit 6,20 g (38,2 mMol) N,N'-Carbonyldiimidazol versetzt und 1 Stunde bei Raumtemperatur gerührt. Zur erhaltenen hellbraunen Lösung gibt man 3,84 g (38 mMol) Triäthylamin und 5,30 g (38 mMol) Sarcosinmethylester-hydrochlorid und rührt das Gemisch während 6 Stunden bei der Siedetemperatur. Nach Entfernen des Dimethylformamids im Vakuum wird der Rückstand aus Äthanol kristallisiert. Man erhält 3,4-Dihydro-4-methyl-2H-pyrido[3,2-e] [1,4]diazepin-2,5(1H)-dion vom Schmelzpunkt 272—274°.

b) 3 g (15,7 mMol) 3,4-Dihydro-4-methyl-2H-pyrido[3,2-e] [1,4]diazepin-2,5(1H)-dion in 20 ml Dimethylformamid versetzt man mit 0,61 g (15,9 mMol) Natriumhydrid (60-proz. Öldispersion), rührt während 1 Stunde bei Raumtemperatur, tropft anschließend bei 20° 2,72 g (15,8 mMol) Diäthylchlorphosphat zu und rührt noch während 20 Minuten bei 20°.

Inzwischen wird eine Lösung von 1,77 g (15,8 mMol) Kalium-t-butylat in 3 ml Dimethylformamid auf —50° abgekühlt und mit 2,28 g (15,8 mMol) Isocyanessigsäure-t-butylester versetzt. Die erhaltene orange Lösung wird bei —10° bis —20° dem obigen Reaktionsgemisch zugetropft. Man entfernt die Kühlung, rührt noch während 20 Minuten, neutralisiert mit Essigsäure, gießt auf Wasser und extrahiert mit Chloroform. Die vereinigten Chloroformauszüge werden mit Wasser gewaschen, über Magnesiumsulfat getrocknet und eingedampft. Durch Säulenchromatographie an Kieselgel und anschließendes Umkristallisieren aus Essigester erhält man t-Butyl-5,6-dihydro-5-methyl-6-oxo-4H-imidazo[1,5-a]pyrido[2,3-f] [1,4]diazepin-3-carboxylat vom Schmelzpunkt 234—236°.

## Beispiel 3

Eine Suspension von 1,51 g (34,5 mMol) Natriumhydrid (55-proz. Öldispersion) in 40 ml trockenem Dimethylformamid wird mit 6,0 g (31,4 mMol) 3,4-Dihydro-4-methyl-2H-pyrido[3,2-e] [1,4]diazepin-2,5(1H)-dion versetzt, während 45 Minuten bei Raumtemperatur gerührt, anschließend auf —35° abgekühlt, mit 5,0 ml (34,5 mMol) Diäthylchlorphosphat tropfenweise versetzt und während 10 Minuten bei —30° bis —15° gerührt.

Inzwischen wird eine Lösung von 3,86 g (34,5 mMol) Kalium-t-butylat in 8 ml trockenem Dimethylformamid in einem Aceton/Trockeneisbad abgekühlt, mit 3,8 ml (34,5 mMol) Isocynessigsäureäthylester versetzt und bei —15° dem obigen Reaktionsgemisch zugetropft. Man entfernt das Kühlbad, rührt während ca. 15 Minuten bei —15° bis 5°, neutralisiert mit Eisessig, gießt auf 150 ml Wasser und extrahiert dreimal mit Chloroform. Die Chloroformauszüge werden einmal mit gesättigter Natriumchloridlösung gewaschen, über Magnesiumsulfat getrocknet und eingedampft. Das erhaltene Rohprodukt wird an Kieselgel unter Eluieren mit Chloroform/Methanol (19 : 1) chromatographiert und anschließend aus Essigester kristallisiert. Man erhält Äthyl-5,6-dihydro-5-methyl-6-oxo-4H-imidazo[1,5-a]pyrido[2,3-f] [1,4]diazepin-3-carboxylat vom Schmelzpunkt 252—254°.

## Beispiel 4

a) Ein Gemisch aus 1,61 g (5,6 mMol) Äthyl-5,6-dihydro-5-methyl-6-oxo-4H-imidazo[1,5-a]pyrido-[2,3-f] [1,4]diazepin-3-carboxylat, 0,33 g (8,2 mMol) Natriumhydroxid, 9 ml Wasser und 27 ml Äthylalkohol wird während 25 Minuten unter Rückfluß zum Sieden erhitzt. Man versetzt mit 8,2 ml 1N Salzsäure, entfernt das Äthanol durch Destillation, verdünnt mit Wasser und läßt während 1 Stunde im Eisbad stehen. Das ausgefallene Material wird abgenutscht, mit Wasser gewaschen und getrocknet.

Man erhält 5,6-Dihydro-5-methyl-6-oxo-4H-imidazo[1,5-a]pyrido[2,3-f] [1,4]diazepin-3-carbonsäure vom Schmelzpunkt 292—295°.

b) 1,0 g (3,9 mMol) 5,6-Dihydro-5-methyl-6-oxo-4H-imidazo[1,5-a]pyrido[2,3-f] [1,4]diazepin-3-carbonsäure werden bis zur Beendigung der Gasentwicklung mit dem Bunsenbrenner erhitzt. Anschließend wird das Rohprodukt unter Eluieren mit Chloroform/Methanol (4 : 1) an Kieselgel chromatographiert und anschließend aus Essigester kristallisiert. Man erhält 4,5-Dihydro-5-methyl-6H-imidazo[1,5-a]pyrido[2,3-f] [1,4]diazepin-6-on vom Schmelzpunkt 199—201°.

Beispiel 5

0,56 g (2,6 mMol) 4,5-Dihydro-5-methyl-6H-imidazo[1,5-a]pyrido[2,3-f] [1,4]diazepin-6-on und 0,35 g (2,6 mMol) N-Chlorsuccinimid werden mit 10 ml Dimethylformamid versetzt und während 30 Minuten bei 90—100° gerührt. Nach Eindampfen zur Trockne wird der Rückstand unter Eluieren mit Chloroform, das 4% Methanol enthält, an Kieselgel chromatographiert. Nach dem Umkristallisieren aus Essigester erhält man 3-Chlor-4,5-dihydro-5-methyl-6H-imidazo[1,5-a]pyrido[2,3-f] [1,4]diazepin-6-on vom Schmelzpunkt 247—248°.

Beispiel 6

a) 12,0 g (73,1 mMol) 3-Azaisatosäureanhydrid und 6,6 g (73,2 mMol) Sarcosin werden in 180 ml Dimethylsulfoxid während 4,25 Stunden bei 100° gerührt. Das Lösungsmittel wird im Hochvakuum entfernt und der ölige Rückstand während etwa 4 Stunden auf 130° erhitzt. Nach dem Abkühlen wird das kristalline Produkt in 150 ml Methanol aufgeschlämmt. Es wird abgenutscht, mit Methanol gewaschen und getrocknet. Man erhält 3,4-Dihydro-4-methyl-2H-pyrido[2,3-e] [1,4]diazepin-2,5(1H)-dion vom Schmelzpunkt 245—247°.

b) Eine Suspension von 1,39 g (31,9 mMol) Natriumhydrid (55-proz. Öldispersion) in 40 ml trockenem Dimethylformamid wird mit 5,3 g (27,7 mMol) 3,4-Dihydro-4-methyl-2H-pyrido[2,3-e] [1,4]diazepin-2,5(1H)-dion versetzt, während 30 Minuten bei Raumtemperatur gerührt, auf —35° abgekühlt, mit 4,8 ml (31,9 mMol) Diäthylchlorphosphat tropfenweise versetzt und während 15 Minuten bei —35° bis —15° gerührt.

Inzwischen wird eine Lösung von 3,7 g (33,2 mMol) Kalium-t-butylat in 10 ml trockenem Dimethylformamid in einem Aceton/Trockeneisbad abgekühlt und mit 4,65 g (33,2 mMol) Isocyanessigsäure-t-butylester versetzt. Die so erhaltene Lösung wird bei —15° zum obigen Reaktionsgemisch getropft. Anschließend entfernt man die Kühlung, rührt noch während 1 Stunde, neutralisiert mit 1,9 ml Eisessig und dampft im Hochvakuum zur Trockne ein. Der Rückstand wird unter Eluieren mit Chloroform/Methanol (19 : 1) an Kieselgel chromatographiert und anschließend aus Essigester umkristallisiert. Man erhält t-Butyl-5,6-dihydro-5-methyl-6-oxo-4H-imidazo[1,5-a]pyrido[3,2-f] [1,4]diazepin-3-carboxylat vom Schmelzpunkt 150—151°.

Beispiel 7

Zu einer gerührten Suspension von 1,81 g (41,5 mMol) Natriumhydrid (55-proz. Öldispersion) in 45 ml trockenem Dimethylformamid gibt man 6,9 g (36,1 mMol) 3,4-Dihydro-4-methyl-2H-pyrido[4,3-e] [1,4]benzodiazepin-2,5(1H)-dion und läßt während 1 Stunde rühren. Anschließend tropft man bei —30° 6,0 ml (41,5 mMol) Diäthylchlorphosphat dazu.

Inzwischen wird eine Lösung von 4,85 g (43,3 mMol) Kalium-t-butylat in 10 ml trockenem Dimethylformamid in einem Aceton/Trockeneisbad abgekühlt, mit 4,8 ml (43,3 mMol) Isocyanessigsäureäthylester versetzt und bei —15° bis —20° dem obigen Reaktionsgemisch zugetropft. Man entfernt das Kühlbad, rührt während 20 Minuten, neutralisiert mit Eisessig, gießt auf 100 ml Wasser und extrahiert sechsmal mit Methylenchlorid. Die Methylenchloridlösung wird über Magnesiumsulfat getrocknet und eingedampft. Das Rohprodukt chromatographiert man an Kieselgel unter Eluieren mit Chloroform/Methanol (19 : 1). Zweimaliges Umkristallisieren des erhaltenen Stoffes aus Essigester liefert Äthyl-5,6-dihydro-5-methyl-6-oxo-4H-imidazo[1,5-a]pyrido[3,4-f] [1,4]diazepin-3-carboxylat vom Schmelzpunkt 205—206°.

t-Butyl-5,6-dihydro-5-methyl-6-oxo-4H-imidazo[1,5-a]pyrido[3,4-f] [1,4]diazepin-3-carboxylat (Wirkstoff A) kann wie in den Beispielen A bis G angegeben als Wirkstoff zur Herstellung von pharmazeutischen Präparaten verwendet werden:

Beispiel A

Es werden in üblicher Weise Tabletten folgender Zusammensetzung hergestellt:

| | mg/Tablette |
|---|---|
| Wirkstoff A | 1 |
| Milchzucker | 103 |
| Maisstärke | 25 |
| Mikrokristalline Cellulose | 70 |
| Magnesiumstearat | 1 |
| Total | 200 |

Beispiel B

Es werden Kapseln folgender Zusammensetzung hergestellt:

| | mg/Kapsel |
|---|---|
| Wirkstoff A | 1 |
| Milchzucker | 164 |
| Maisstärke | 30 |
| Talk | 5 |
| Total | 200 |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

Beispiel C

Es werden Injektionslösungen folgender Zusammensetzung hergestellt:

| | pro ml |
|---|---|
| Wirkstoff A | 2,5 mg |
| D-(—)-Mannit | 52 mg |
| Wasser für Injektion q. s. ad | 1,0 ml |

Zur Herstellung von 10 000 ml Injektionslösung löst man 520 g D-(—)-Mannit in 9000 ml Wasser (für Injektion). Dann löst man 25 g des Wirkstoffs und ergänzt mit Wasser (für Injektion) auf 10 000 ml. Diese Lösung wird filtriert und in Ampullen geeigneter Größe abgefüllt; man füllt das Restvolumen der Ampullen mit Stickstoff, schmilzt die Ampullen zu und sterilisiert sie z. B. während 20 Minuten bei 120°.

Beispiel D

Es werden Suppositorien folgender Zusammensetzung hergestellt:

| | g/Supp. |
|---|---|
| Wirkstoff A | 0,001 |
| Cacaobutter (Smp. 36—37°) | 1,255 |
| Carnauba-Wachs | 0,044 |
| Total | 1,3 |

Cacaobutter und Carnauba-Wachs werden in einem Glas- oder Stahlgefäß geschmolzen, gründlich vermengt und auf 45° abgekühlt. Hierauf gibt man den fein pulverisierten Wirkstoff zu und rührt, bis es vollständig dispergiert ist. Man gießt die Mischung in Suppositorienformen geeigneter Größe, läßt abkühlen, nimmt dann die Suppositorien aus den Formen und verpackt sie einzeln in Wachspapier oder Metallfolien.

## Beispiel E

Es werden Kapseln folgender Zusammensetzung hergestellt:

| | mg/Kapsel |
|---|---|
| Wirkstoff A | 20,0 |
| (+)-5-(o-Chlorphenyl)-1,3-dihydro-3-methyl-7-nitro-2H-1,4-benzodiazepin-2-on (Wirkstoff B) | 30,0 |
| Milchzucker krist. | 100,0 |
| Maisstärke weiß | 27,5 |
| Talk | 10,0 |
| Magnesiumstearat | 2,5 |
| Total | 190,0 |

Die beiden Wirkstoffe werden mit den Hilfsstoffen gut vermischt und zu je 190,0 mg in Steckkapseln geeigneter Größe abgefüllt.

## Beispiel F

Es werden Tabletten folgender Zusammensetzung hergestellt:

| | mg/Tablette |
|---|---|
| Wirkstoff A | 10,0 |
| (+)-5-(o-Chlorphenyl)-1,3-dihydro-3-methyl-7-nitro-2H-1,4-benzodiazepin-2-on (Wirkstoff B) | 30,0 |
| Milchzucker pulvis | 15,0 |
| Maisstärke weiß | 19,5 |
| Povidon K30 | 3,5 |
| Maisstärke weiß | 10,0 |
| Magnesiumstearat | 2,0 |
| Total | 90,0 |

Die beiden Wirkstoffe, Milchzucker pulvis und die erste Portion Maisstärke weiß werden gemischt und gesiebt. Diese Mischung wird mit einer Lösung von Povidon K30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 90 mg schweren Tabletten verpreßt.

## Beispiel G

Es werden Tabletten folgender Zusammensetzung hergestellt:

| | mg/Tablette |
|---|---|
| Wirkstoff A | 30 |
| (+)-5-(o-Chlorphenyl)-1,3-dihydro-3-methyl-7-nitro-2H-1,4-benzodiazepin-2-on (Wirkstoff B) | 30 |
| Milchzucker pulvis | 22 |
| Maisstärke weiß | 22 |
| Povidon K30 | 6 |
| Maisstärke weiß | 16 |
| Magnesiumstearat | 4 |
| Total | 130 |

Die beiden Wirkstoffe, Milchzucker pulvis und die erste Portion Maisstärke weiß werden gemischt und gesiebt. Diese Mischung wird mit einer Lösung von Povidon K30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 130 mg schweren Tabletten verpreßt.

**Patentansprüche**

1. Imidazodiazepine der allgemeinen Formel

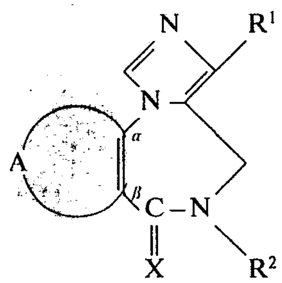

(I)

worin A zusammen mit den beiden $\alpha$ und $\beta$ bezeichneten Kohlenstoffatomen einen Pyridinring, X ein Sauerstoff- oder Schwefelatom, $R^1$ Wasserstoff, Halogen, $(C_{1-7})$-Alkyl, $(C_{1-7})$-Alkoxymethyl oder die Gruppe $-COOR^3$ und $R^2$ und $R^3$ je $(C_{1-7})$-Alkyl bedeuten,
und pharmazeutisch annehmbare Säureadditionssalze davon.

2. Verbindungen gemäß Anspruch 1, dadurch gekennzeichnet, daß $R^1$ Chlor oder die Gruppe $-COOR^3$ und $R^3$ Methyl, Äthyl, Isopropyl oder t-Butyl bedeuten.

3. Verbindungen gemäß Anspruch 1 oder 2, dadurch gekennzeichnet, daß $R^1$ die Gruppe $-COOR^3$ und $R^3$ Äthyl oder t-Butyl bedeuten.

4. Verbindungen gemäß einem der Ansprüche 1 bis 3, dadurch gekennzeichnet, daß A zusammen mit den beiden mit $\alpha$ und $\beta$ bezeichneten Kohlenstoffatomen die Gruppe

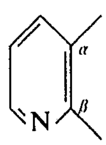 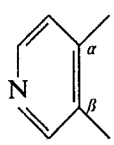 oder 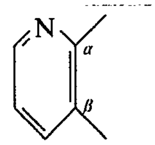

(a) (b) (d)

bedeutet.

5. Verbindungen gemäß Anspruch 4, dadurch gekennzeichnet, daß A die in Anspruch 4 definierte Gruppe (b) bedeutet.

6. Verbindungen gemäß einem der Ansprüche 1 bis 5, dadurch gekennzeichnet, daß $R^2$ Methyl bedeutet.

7. Verbindungen gemäß einem der Ansprüche 1 bis 6, dadurch gekennzeichnet, daß X ein Sauerstoffatom bedeutet.

8. t-Butyl-5,6-dihydro-5-methyl-6-oxo-4H-imidazo[1,5-a]pyrido[3,4-f] [1,4]diazepin-3-carboxylat.

9. Äthyl-5,6-dihydro-5-methyl-6-oxo-4H-imidazo[1,5-a]pyrido[3,4-f] [1,4]diazepin-3-carboxylat.

10. t-Butyl-5,6-dihydro-5-methyl-6-oxo-4H-imidazo[1,5-a]pyrido[2,3-f] [1,4]diazepin-3-carboxylat,
Äthyl-5,6-dihydro-5-methyl-6-oxo-4H-imidazo[1,5-a]pyrido[2,3-f] [1,4]diazepin-3-carboxylat,
3-Chlor-4,5-dihydro-5-methyl-6H-imidazo[1,5-a]pyrido[2,3-f] [1,4]diazepin-6-on
und
t-Butyl-5,6-dihydro-5-methyl-6-oxo-4H-imidazo[1,5-a]pyrido[3,2-f] [1,4]diazepin-3-carboxylat.

11. Verbindungen der allgemeinen Formel

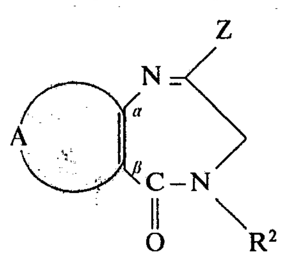

(II)

worin A zusammen mit den beiden mit $\alpha$ und $\beta$ bezeichneten Kohlenstoffatomen einen Pyridinring, $R^2$ $(C_{1-7})$-Alkyl und Z eine Abgangsgruppe bedeuten.

12. Verbindungen der allgemeinen Formel

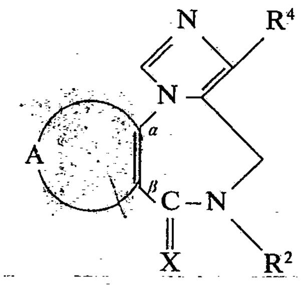

(XIV)

worin A zusammen mit den beiden mit α und β bezeichneten Kohlenstoffatomen einen Pyridinring, $R^2$ $(C_{1-7})$-Alkyl, X ein Sauerstoff- oder Schwefelatom, $R^4$ die Gruppe $-COOH$, $-CH_2-OH$ oder $-CH(R^5)-Z'$, $R^5$ Wasserstoff oder $(C_{1-6})$-Alkyl und Z' eine Abgangsgruppe bedeuten.

13. Verbindungen gemäß einem der Ansprüche 1 bis 10 als pharmazeutische Wirkstoffe.

14. Pharmazeutische Wirkstoffe gemäß Anspruch 13, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

15. Verfahren zur Herstellung von Verbindungen gemäß einem der Ansprüche 1 bis 10, dadurch gekennzeichnet, daß man

a) eine Verbindung der allgemeinen Formel

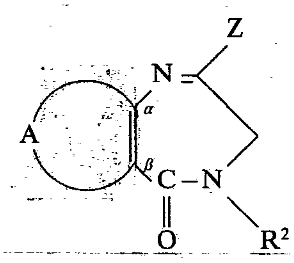

(II)

worin A und $R^2$ die in Anspruch 1 angegebene Bedeutung besitzen, und Z eine Abgangsgruppe bedeutet,
in Gegenwart einer Base mit einem Isocyanessigester der allgemeinen Formel

$$CN-CH_2-COOR^3 \qquad \text{(III)}$$

worin $R^3$ die in Anspruch 1 angegebene Bedeutung besitzt,
umsetzt, oder

b) eine Carbonsäure der allgemeinen Formel

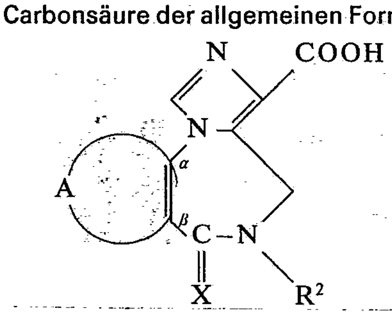

(IV)

worin A, X und $R^2$ die in Anspruch 1 angegebene Bedeutung besitzen,
decarboxyliert, oder

c) eine Verbindung der allgemeinen Formel

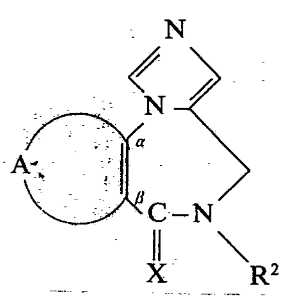

(Ia)

worin A, X und $R^2$ die in Anspruch 1 angegebene Bedeutung besitzen, halogeniert, oder

d) eine Verbindung der allgemeinen Formel

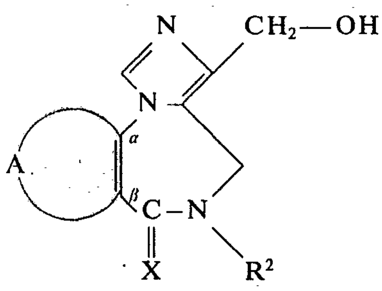

(Va)

bzw.

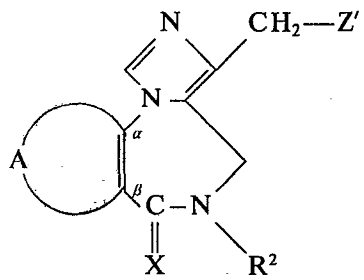

(VIa)

worin A, X und $R^2$ die in Anspruch 1 angegebene Bedeutung besitzen und Z' eine Abgangsgruppe bedeutet, mit einem einen ($C_{1-7}$)-Alkylrest liefernden Alkylierungsmittel, bzw. einem ($C_{1-7}$)-Alkohol veräthert, oder

e) in einer Verbindung der allgemeinen Formel

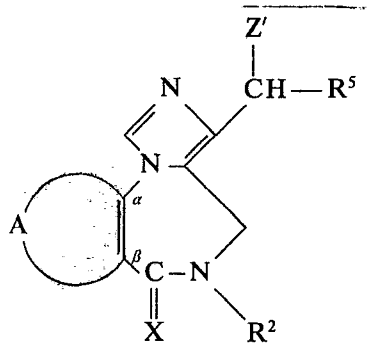

(VI)

worin A, X und $R^2$ die in Anspruch 1 angegebene und Z' obige Bedeutung besitzen und $R^5$ Wasserstoff oder ($C_{1-6}$)-Alkyl bedeutet, die mit Z' bezeichnete Abgangsgruppe unter reduktiven Bedingungen abspaltet, oder

f) eine Verbindung der allgemeinen Formel

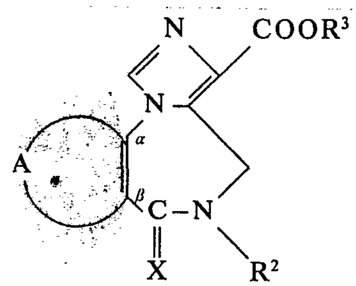

(Ic)

worin A, X, $R^2$ und $R^3$ die in Anspruch 1 angegebene Bedeutung besitzen, umestert, oder

g) in einer Verbindung der allgemeinen Formel

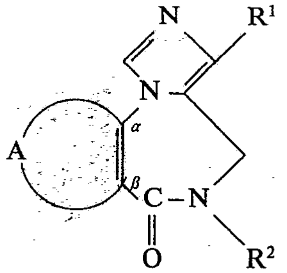

(Ib)

worin A, $R^1$ und $R^2$ die in Anspruch 1 angegebene Bedeutung besitzen, die Carbonylgruppe in die Thiocarbonylgruppe überführt, und

h) erwünschtenfalls eine erhaltene Verbindung der allgemeinen Formel I in ein pharmazeutisch annehmbares Säureadditionssalz überführt.

16. Arzneimittel, enthaltend eine Verbindung gemäß einem der Ansprüche 1 bis 10.

17. Arzneimittel gemäß Anspruch 16, welche die zentral dämpfenden, muskelrelaxierenden, ataktischen, blutdrucksenkenden und atemdepressiven Eigenschaften von tranquilisierend wirksamen 1,4-Benzodiazepinen antagonisieren.

18. Schistosomizid wirksames Arzneimittel, enthaltend eine Verbindung gemäß einem der Ansprüche 1 bis 10 und (+)-5-(o-Chlorphenyl)-1,3-dihydro-3-methyl-7-nitro-2H-1,4-benzodiazepin-2-on.

**Claims**

1. Imidazodiazepines of the general formula

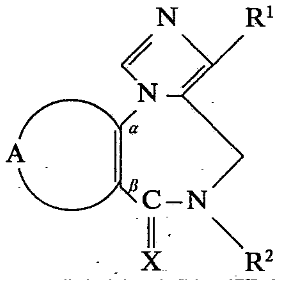

(I)

wherein A together with the two carbon atoms denoted as α and β signifies a pyridine ring, X signifies an oxygen or sulphur atom, $R^1$ signifies hydrogen, halogen, $(C_{1-7})$-alkyl, $(C_{1-7})$-alkoxymethyl or the group $-COOR^3$ and $R^2$ and $R^3$ each signify $(C_{1-7})$-alkyl,
and pharmaceutically acceptable acid addition salts thereof.

2. Compounds according to claim 1, characterized in that $R^1$ signifies chlorine or the group $-COOR^3$ and $R^3$ signifies methyl, ethyl, isopropyl or t-butyl.

3. Compounds according to claim 1 or 2, characterized in that $R^1$ signifies the group $-COOR^3$ and $R^3$ signifies ethyl or t-butyl.

4. Compounds according to any one of claims 1 to 3, characterized in that A together with the two carbon atoms denoted as α and β signifies the group

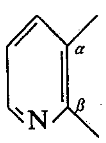

(a)

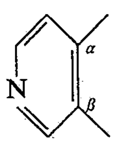

(b)

or

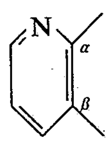

(d)

5. Compounds according to claim 4, characterized in that A signifies the group (b) defined in claim 4.

6. Compounds according to any one of claims 1 to 5, characterized in that $R^2$ signifies methyl.

7. Compounds according to any one of claims 1 to 6, characterized in that X signifies an oxygen atom.

8. t-Butyl 5,6-dihydro-5-methyl-6-oxo-4H-imidazo[1,5-a]pyrido[3,4-f] [1,4]diazepine-3-carboxylate.

9. Ethyl 5,6-dihydro-5-methyl-6-oxo-4H-imidazo[1,5-a]pyrido[3,4-f] [1,4]diazepine-3-carboxylate.

10. t-Butyl 5,6-dihydro-5-methyl-6-oxo-4H-imidazo[1,5-a]pyrido[2,3-f] [1,4]diazepine-3-carboxylate,
ethyl 5,6-dihydro-5-methyl-6-oxo-4H-imidazo[1,5-a]pyrido[2,3-f] [1,4]diazepine-3-carboxylate,
3-chloro-4,5-dihydro-5-methyl-6H-imidazo[1,5-a]pyrido[2,3-f] [1,4]diazepin-6-one and
t-butyl 5,6-dihydro-5-methyl-6-oxo-4H-imidazo[1,5-a]pyrido[3,2-f] [1,4]diazepine-3-carboxylate.

11. Compounds of the general formula

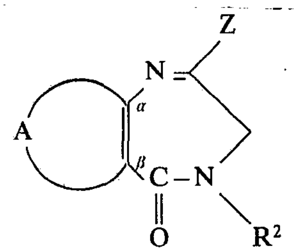

(II)

wherein A together with the two carbon atoms denoted as $\alpha$ and $\beta$ signifies a pyridine ring, $R^2$ signifies $(C_{1-7})$-alkyl and Z signifies a leaving group.

12. Compounds of the general formula

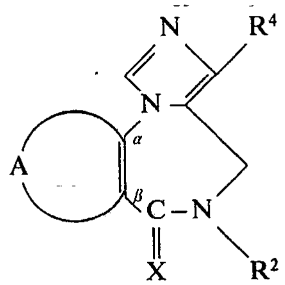

(XIV)

wherein A together with the two carbon atoms denoted as $\alpha$ and $\beta$ signifies a pyridine ring, $R^2$ signifies $(C_{1-7})$-alkyl, X signifies an oxygen or sulphur atom, $R^4$ signifies the group $-COOH$, $-CH_2-OH$ or $-CH(R^5)-Z'$, $R^5$ signifies hydrogen or $(C_{1-6})$-alkyl and Z' signifies a leaving group.

13. Compounds according to any one of claims 1 to 10 as pharmaceutically active substances.

14. Pharmaceutically active substances according to claim 13, which antagonize the central-depressant, muscle relaxant, ataxic, blood pressure-lowering and respiratory-depressant properties of 1,4-benzodiazepines which have tranquillizing activity.

15. A process for the manufacture of compounds according to any one of claims 1 to 10, characterized by

a) reacting a compound of the general formula

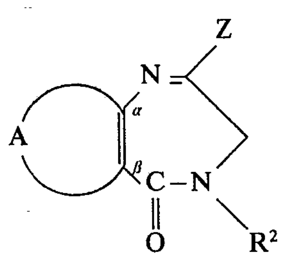

(II)

wherein A and $R^2$ have the significance given in claim 1, and Z signifies a leaving group, in the presence of a base with an isocyanoacetic ester of the general formula

$$CN-CH_2-COOR^3 \quad (III)$$

wherein $R^3$ has the significance given in claim 1,
or

b) decarboxylating a carboxylic acid of the general formula

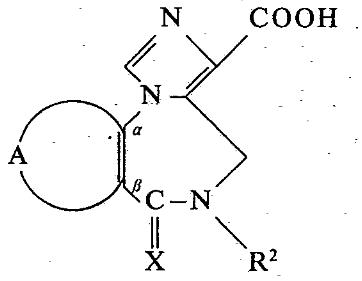

(IV)

wherein A, X and $R^2$ have the significance given in claim 1, or

c) halogenating a compound of the general formula

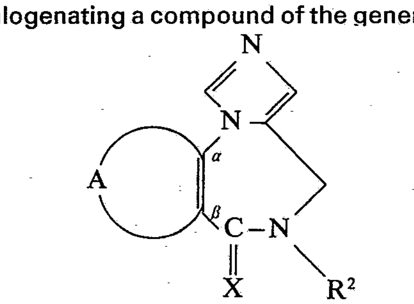

(Ia)

wherein A, X and $R^2$ have the significance given in claim 1, or

d) etherifying a compound of the general formula

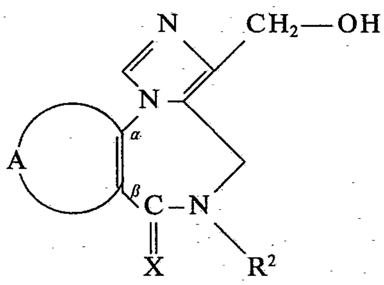

(Va)

or

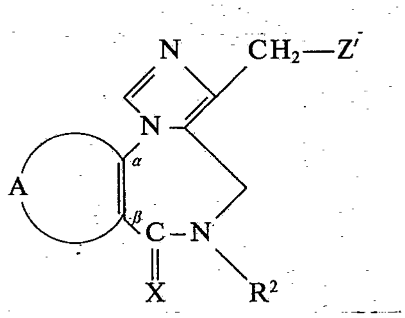

(VIa)

wherein A, X and $R^2$ have the significance given in claim 1 and Z′ signifies a leaving group, with an alkylating agent yielding a ($C_{1-7}$)-alkyl residue and a ($C_{1-7}$)-alcohol, respectively, or

e) cleaving off under reductive conditions the leaving group denoted by Z′ in a compound of the general formula

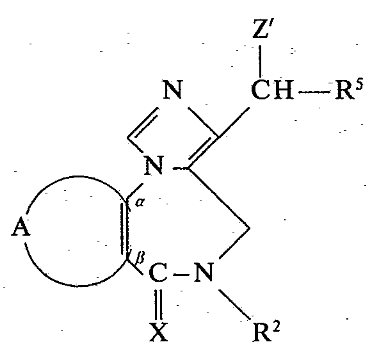

(VI)

wherein A, X and $R^2$ have the significance given in claim 1 and Z′ has the above significance and $R^5$ signifies hydrogen or $(C_{1-6})$-alkyl,
or

f) trans-esterifying a compound of the general formula

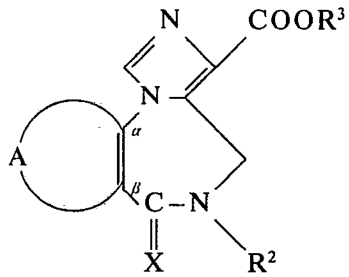

(Ic)

wherein A, X, $R^2$ and $R^3$ have the significance given in claim 1,
or

g) converting the carbonyl group in a compound of the general formula

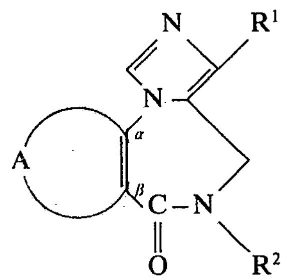

(Ib)

wherein A, $R^1$ and $R^2$ have the significance given in claim 1,
into the thiocarbonyl group, and

h) if desired, converting a compound of general formula I obtained into a pharmaceutically acceptable acid addition salt.

16. A medicament containing a compound according to any one of claims 1 to 10.

17. A medicament according to claim 16, which antagonizes the central-depressant, muscle relaxant, ataxic, blood pressure-lowering and respiratory-depressant properties of 1,4-benzodiazepines which have tranquillizing activity.

18. A schistosomicidally-active medicament containing a compound according to any one of claims 1 to 10 and (+)-5-(o-chlorophenyl)-1,3-dihydro-3-methyl-7-nitro-2H-1,4-benzodiazepin-2-one.

**Revendications**

1. Imidazodiazépines de formule générale

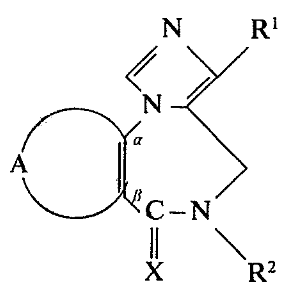

(I)

dans laquelle A forme avec les deux atomes de carbone marqués α et β un cycle de pyridine, X représente un atome d'oxygène ou de soufre, $R^1$ représente l'hydrogène, un halogène, un groupe alkyle en $C_{1-7}$, alcoxy-en $C_{1-7}$-méthyle ou le groupe de formule $-COOR^3$, $R^2$ et $R^3$ représentant chacun un groupe alkyle en $C_{1-7}$, et leurs sels formés par addition avec des acides acceptables pour l'usage pharmaceutique.

2. Composés selon la revendication 1, caractérisés en ce que $R^1$ représente le chlore ou le groupe de formule $-COOR^3$, et $R^3$ représente un groupe méthyle, éthyle, isopropyle ou t-butyle.

3. Composés selon la revendication 1 ou 2, caractérisés en ce que $R^1$ représente le groupe $-COOR^3$ et $R^3$ représente un groupe éthyle ou t-butyle.

4. Composés selon l'une des revendications 1 à 3, caractérisés en ce que A forme avec les deux atomes de carbone marqués $\alpha$ et $\beta$ le groupe

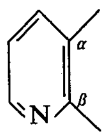 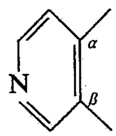 ou 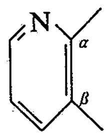

(a) (b) (d)

5. Composés selon la revendication 4, caractérisés en ce que A représente le groupe (b) défini dans la revendication 4.

6. Composés selon l'une des revendications 1 à 5, caractérisés en ce que $R^2$ représente un groupe méthyle.

7. Composés selon l'une des revendications 1 à 6, caractérisés en ce que X représente un atome d'oxygène.

8. Le 5,6-dihydro-5-méthyl-6-oxo-4H-imidazo[1,5-a]pyrido[3,4-f] [1,4]diazépine-3-carboxylate de t-butyle.

9. Le 5,6-dihydro-5-méthyl-6-oxo-4H-imidazo[1,5-a]pyrido[3,4-f] [1,4]diazépine-3-carboxylate d'éthyle.

10. Le 5,6-dihydro-5-méthyl-6-oxo-4H-imidazo[1,5-a]pyrido[2,3-f] [1,4]diazépine-3-carboxylate de t-butyle,
le 5,6-dihydro-5-méthyl-6-oxo-4H-imidazo[1,5-a]pyrido[2,3-f] [1,4]diazépine-3-carboxylate d'éthyle,
la 3-chloro-4,5-dihydro-5-méthyl-6H-imidazo[1,5-a]pyrido[2,3-f] [1,4]diazépine-6-one
et
le 5,6-dihydro-5-méthyl-6-oxo-4H-imidazo[1,5-a]pyrido[2,3-f] [1,4]diazépine-3-carboxylate de t-butyle.

11. Composés de formule générale

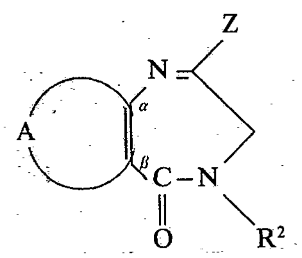

(II)

dans laquelle A forme avec les deux atomes de carbone marqués $\alpha$ et $\beta$ un cycle de pyridine, $R^2$ représente le groupe alkyle en $C_{1-7}$ et Z représente un groupe éliminable.

12. Composés de formule générale:

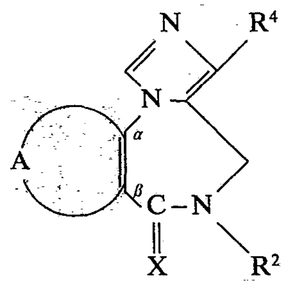

(XIV)

dans laquelle A forme avec les deux atomes de carbone marqués $\alpha$ et $\beta$ un cycle de pyridine, $R^2$ représente un groupe alkyle en $C_{1-7}$, X représente un atome d'oxygène ou de soufre, $R^4$ représente un groupe $-COOH$, $-CH_2OH$ ou $-CH(R^5)-Z'$, $R^5$ représente l'hydrogène ou un groupe alkyle en $C_{1-6}$ et Z' représente un groupe éliminable.

13. Composés selon l'une des revendications 1 à 10, en tant que substances actives pharmaceutiques.

14. Substances actives pharmaceutiques selon la revendication 13 antagonisant les propriétés de sédation centrale, de relaxation musculaire, d'ataxie, d'hypotension et de dépression respiratoire des 1,4-benzodiazépine à effet tranquillisant.

15. Procédé de préparation des composés selon l'une des revendications 1 à 10, caractérisé en ce que:

(a) on fait réagir un composé de formule générale:

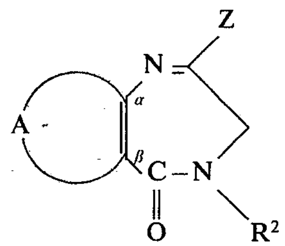

(II)

dans laquelle A et $R^2$ ont les significations indiquées dans la revendication 1 et Z représente un groupe éliminable,
en présence d'une base avec un ester isocyanacétique de formule générale:

$$CN-CH_2-COOR^3 \quad (III)$$

dans laquelle $R^3$ a la signification indiquée dans la revendication 1, ou bien

(b) on décarboxyle un acide carboxylique de formule générale:

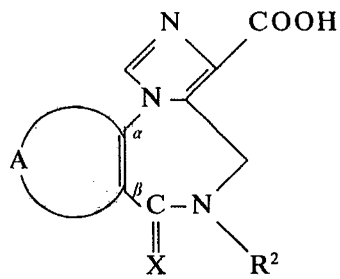

(IV)

dans laquelle A, X et $R^2$ ont les significations indiquées dans la revendication 1, ou bien

(c) on halogène un composé de formule générale:

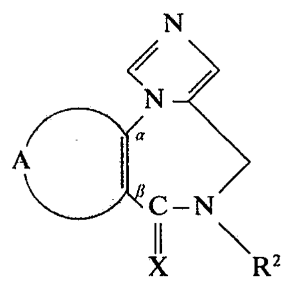

(Ia)

dans laquelle A, X et $R^2$ ont les significations indiquées dans la revendication 1, ou bien

(d) on éthérifie un composé de formule générale respective:

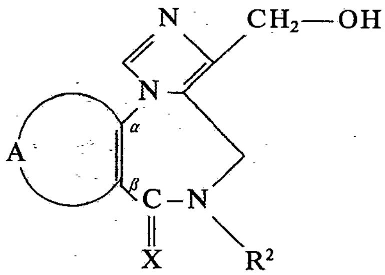

(Va)

ou

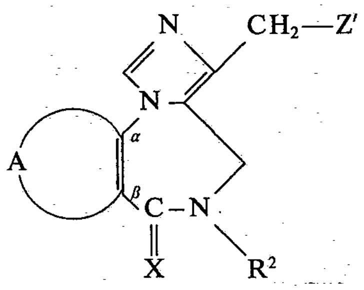

(VIa)

dans laquelle A, X et $R^2$ ont les significations indiquées dans la revendication 1 et Z' représente un groupe éliminable,
par un agent alkylant apportant un groupe alkyle en $C_{1-7}$ ou par un alcool en $C_{1-7}$ respectivement, ou bien

(e) dans un composé de formule générale:

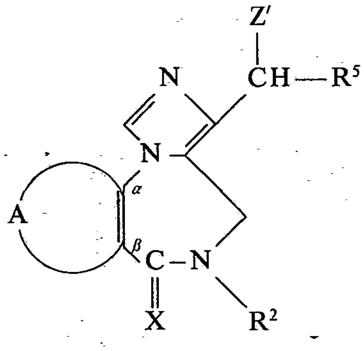

(VI)

dans laquelle A, X et $R^2$ ont les significations indiquées dans la revendication 1 et Z' a la signification indiquée ci-dessus, $R^5$ représentant l'hydrogène ou un groupe alkyle en $C_{1-6}$,
on élimine dans des conditions réductrices le groupe éliminable représenté par Z',
ou bien

(f) on transestérifie un composé de formule générale

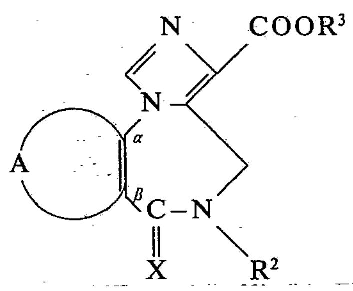

(Ic)

dans laquelle A, X, $R^2$ et $R^3$ ont les significations indiquées dans la revendication 1, ou bien

(g) dans un composé de formule générale:

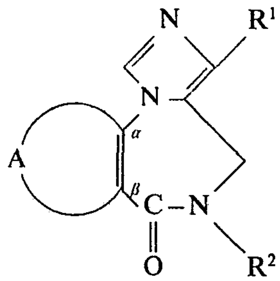

(Ib)

dans laquelle A, $R^1$ et $R^2$ ont les significations indiquées dans la revendication 1, on convertit le groupe carbonyle en un groupe thiocarbonyle, et

(h) si on le désire, on convertit un composé obtenu de formule générale I en un sel formé par addition avec un acide acceptable pour l'usage pharmaceutique.

16. Médicament contenant un composé selon l'une des revendications 1 à 10.

17. Médicament selon la revendication 16, antagonisant les propriétés de sédation centrale, de relaxation musculaire, d'ataxie, d'hypotension et de dépression respiratoire des 1,4-benzodiazépines à effet tranquillisant.

18. Médicament à activité schistosomicide, contenant un composé selon l'une des revendications 1 à 10 et de la (+)-5-(o-chlorophényl)-1,3-dihydro-3-méthyl-7-nitro-2H-1,4-benzodiazépine-2-one.